# EUROPEAN PATENT SPECIFICATION

(11) **EP 4 032 488 B1**
(45) Date of publication and mention of the grant of the patent: **24.04.2024**
(21) Application number: 20879506.2
(22) Date of filing: 14.10.2020
(51) Int. Cl.: A61B 17/128, A61B 17/00

(54) **SURGICAL APPARATUS**
CHIRURGISCHE VORRICHTUNG
APPAREIL CHIRURGICAL

(30) Priority: 23.10.2019 CN 201911012319; 23.10.2019 CN 201921787990 U; 23.10.2019 CN 201911012988
(43) Date of publication of application: 27.07.2022
(73) Proprietor: IntoCare Medical Technology (Suzhou) Co., Ltd., Suzhou, Jiangsu 215000 (CN)
(72) Inventor: ZHANG, Hui, Suzhou, Jiangsu 215000 (CN); XU, Ronghua, Suzhou, Jiangsu 215000 (CN); YANG, Bin, Suzhou, Jiangsu 215000 (CN)
(74) Representative: Gulde & Partner
(86) International application number: PCT/CN2020/120811
(87) International publication number: WO 2021/078045

(56) References cited:
- EP-A2- 3 505 076
- WO-A1-2019/089423
- WO-A1-2019/136604
- CN-A- 101 495 046
- CN-A- 108 348 259
- CN-A- 108 348 259
- CN-A- 108 348 261
- CN-U- 203 436 360
- CN-U- 204 428 101
- CN-U- 211 022 895
- US-A1- 2018 242 977

## Description

### CROSS-REFERENCE TO RELATED APPLICATIONS

The present application is based on and claims the priority of Chinese patent application No.201911012319.5 entitled "surgical apparatus" filed on October 23, 2019, the priority of Chinese patent application No.201921787990.2 entitled "surgical apparatus" filed on October 23, 2019, and the priority of Chinese patent application No.201911012988.2 entitled "medical apparatus and automatic clip-delivery method" filed on October 23, 2019.

### TECHNICAL FIELD

Embodiments of the present disclosure relate to a surgical apparatus and an automatic clip-delivery method thereof.

### BACKGROUND

In order to fully expose a surgical visual field during a surgery, a ligation of vessels around a target tissue is required so as to prevent bleeding. Hemostasis technique has become one of the cores of basic operating techniques in a surgery, and a surgical operation for almost any part of a human body involves bleeding and hemostasis. US2018/242977A1 directs to a reposable endoscopic surgical clip applier includes a handle assembly (100) configured to releasably engage at least two different endoscopic assemblies (200,300,400). The handle assembly (100) is configured such that a ratcheting function thereof is disabled upon engagement of an endoscopic assembly (300) therewith that is not intended for ratcheting use and such that the ratcheting function is enabled upon engagement of an endoscopic assembly (400) therewith that is intended for ratcheting use. Endoscopic assemblies (200,300,400) for use with the handle assembly (100) are also provided. EP3505076A2 directs to a surgical instrument comprising a control system and a strain gage circuit. The operation of the control system is modifiable by an input from the strain gage circuit.

### SUMMARY

It is an objective of the present disclosure to provide a surgical apparatus.

The objective is achieved by the respective independent claim. Further embodiments are defined in the corresponding dependent claims.

### BRIEF DESCRIPTION OF THE DRAWINGS

In order to clearly illustrate the technical solutions of the embodiments of the present disclosure, the drawings of the embodiments will be briefly described in the following; it is obvious that the described drawings are only related to some embodiments of the present disclosure and thus are not limitative to the present disclosure.
Fig. 1 is a schematic structural diagram of a surgical apparatus provided in accordance with an embodiment of the present disclosure;
Fig. 2 is an exploded schematic view of a surgical apparatus provided in accordance with an embodiment of the present disclosure;
Fig. 3 is a schematic structural diagram of a working component and a rotary head provided in accordance with an embodiment of the present disclosure;
Fig. 4 is a schematic cross-sectional view of Fig. 3;
Fig. 5 is a schematic structural diagram of a shaft assembly provided in accordance with an embodiment of the present disclosure;
Fig. 6 is a schematic cross-sectional view of Fig. 5;
Fig. 7 is an exploded schematic view of a shaft assembly provided in accordance with an embodiment of the present disclosure;
Fig. 8 is an exploded schematic view of a coupling component and a shaft assembly provided in accordance with an embodiment of the present disclosure;
Fig. 9 is a partially exploded schematic view of a coupling component provided in accordance with an embodiment of the present disclosure;
Fig. 10 is a schematic structural diagram of a surgical apparatus provided in accordance with another embodiment of the present disclosure;
Fig. 11 is an exploded schematic view of a surgical apparatus provided in accordance with another embodiment of the present disclosure;
Fig. 12 is a schematic cross-sectional view of a working component provided in accordance with another embodiment of the present disclosure;
Fig. 13 is a schematic structural diagram of a shaft assembly provided in accordance with another embodiment of the present disclosure;
Fig. 14 is a schematic structural diagram of a clip-cartridge assembly provided in accordance with another embodiment of the present disclosure;
Fig. 15 is an exploded schematic view of a working component provided in accordance with another embodiment of the present disclosure;
Fig. 16A is a block diagram of an electrical connection relationship between components of a surgical apparatus during automatically delivering a ligating clip according to another embodiment of the present disclosure;
Fig. 16B is a block diagram of an electrical connection relationship between components of a surgical apparatus during firing a ligating clip according to another embodiment of the present disclosure; and
Fig. 17 is a flowchart of an automatic clip-delivery method provided in accordance with yet another embodiment of the present disclosure.

### DETAILED DESCRIPTION

In order to make objects, technical details and advantages of the embodiments of the disclosure apparent, the technical solutions of the embodiments will be described in a clearly and fully understandable way in connection with the drawings related to the embodiments of the disclosure. Apparently, the described embodiments are just a part but not all of the embodiments of the disclosure. Based on the described embodiments herein, those skilled in the art can obtain other embodiment(s), without any inventive work, which should be within the scope of the disclosure.

Unless otherwise defined, all the technical and scientific terms used herein have the same meanings as commonly understood by one of ordinary skill in the art to which the present disclosure belongs. The terms "first," "second," etc., which are used in the present disclosure, are not intended to indicate any sequence, amount or importance, but distinguish various components. Also, the terms "comprise," "comprising," "include," "including," etc., are intended to specify that the elements or the objects stated before these terms encompass the elements or the objects and equivalents thereof listed after these terms, but do not preclude the other elements or objects. The phrases "connect", "connected", etc., are not intended to define a physical connection or mechanical connection, but may include an electrical connection, directly or indirectly. "On," "under," "right," "left" and the like are only used to indicate relative position relationship, and when the position of the object which is described is changed, the relative position relationship may be changed accordingly.

In order to achieve the purpose of stopping bleeding, a ligating clip of a surgical apparatus is generally used for hemostasis. The surgical apparatus includes a reusable handheld component, a working component and a disposable shaft assembly or similar devices. In the case that the handheld component is electrically driven, the shaft assembly is connected with the working component through electric interfaces. However, the electric interface is generally configured in a shape difficult to be coupled, which makes it hard to achieve the coupling between the shaft assembly and the working component and affects the usage thereof.

Embodiments of the present disclosure provide a surgical apparatus including an adapter and a shaft assembly. The adapter includes: a casing having an opening provided at a distal end of the casing; and a rotary head sleeved onto the opening of the casing and configured to be rotatable relative to the casing. The rotary head includes a first through hole and a first guide member provided in the first through hole. The shaft assembly is detachably connected to the adapter, and includes a firing sleeve and a coupling component. The coupling component is disposed at an end of the firing sleeve, and is inserted into the opening of the casing after passing through the first through hole of the rotary head. The coupling component includes a coupling casing and a second guide member disposed on the coupling casing. The first guide member and the second guide member are engaged with each other in a snap-fit manner to keep the rotary head and the shaft assembly in a locked position.

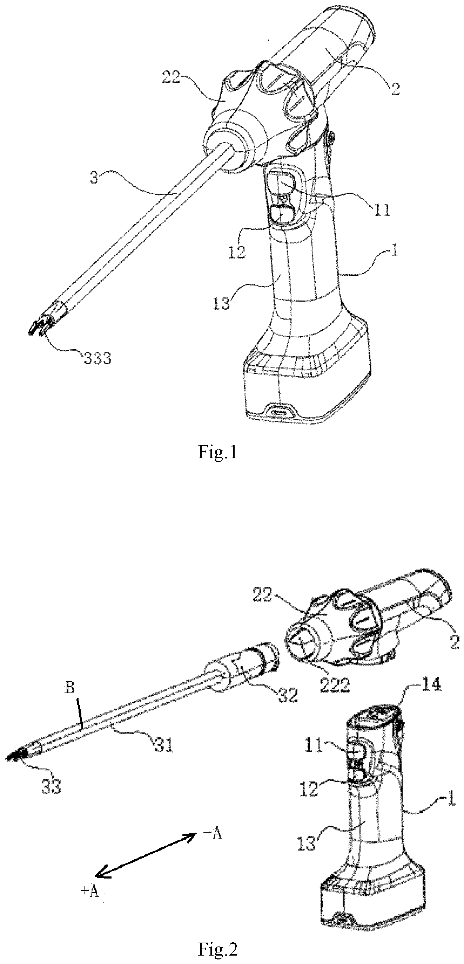

The surgical apparatus according to the embodiments of the present disclosure is described in further details below with reference to the accompanying drawings. Fig. 1 is a schematic structural diagram of a surgical apparatus provided in accordance with an embodiment of the present disclosure. Fig. 2 is an exploded schematic view of a surgical apparatus provided in accordance with an embodiment of the present disclosure.

As shown in Figs. 1 and 2, a surgical apparatus in an embodiment of the present disclosure includes an adapter. The adapter includes a casing having an opening 211 provided at a distal end of the casing. The term "distal end" as used in the present disclosure refers to an end far away from the adapter (including a working component and a handheld component), and may also be understood as an end close to a patient. The term "proximal end" refers to an end close to the adapter (including the working component and the handheld component), and may also be understood as an end far away from the patient. In an embodiment of the present disclosure, the adapter is a handheld device, which may be an integral structure or a split-type structure (i.e., including a plurality of detachable components). In the case that the split-type structure is adopted, the components can be replaced individually according to the application scenario, and thus it is preferable. The embodiment of the present disclosure is described with reference to the case that the adapter is a split-type structure by way of example.

For example, the adapter includes a handheld component 1, and a working component 2 detachably connected to the handheld component 1. The surgical apparatus further includes a shaft assembly 3. The shaft assembly 3 is detachably connected to the working component 2. Because the connection between the three components, i.e., the handheld component 1, the working component 2 and the shaft assembly 3, is detachable, any one of the handheld component 1, the working component 2 and the shaft assembly 3 is allowed to be directly replaced in case of damage without the need of replacing the entire surgical apparatus, so that the medical cost is saved and the economic burden of the patient is reduced.

The working component 2 and the handheld component 1 are detachably connected with each other in a snap-fit matter, in a threaded manner, in a plugged-in manner and the like. The working component 2 and the shaft assembly 3 are detachably connected with each other in a snap-fit matter, in a threaded manner, in a plugged-in manner and the like. In the present embodiment, the working component 2 is connected with the handheld component 1 in a snap-fit matter, and the working component 2 is connected with the shaft assembly 3 in a plugged-in manner.

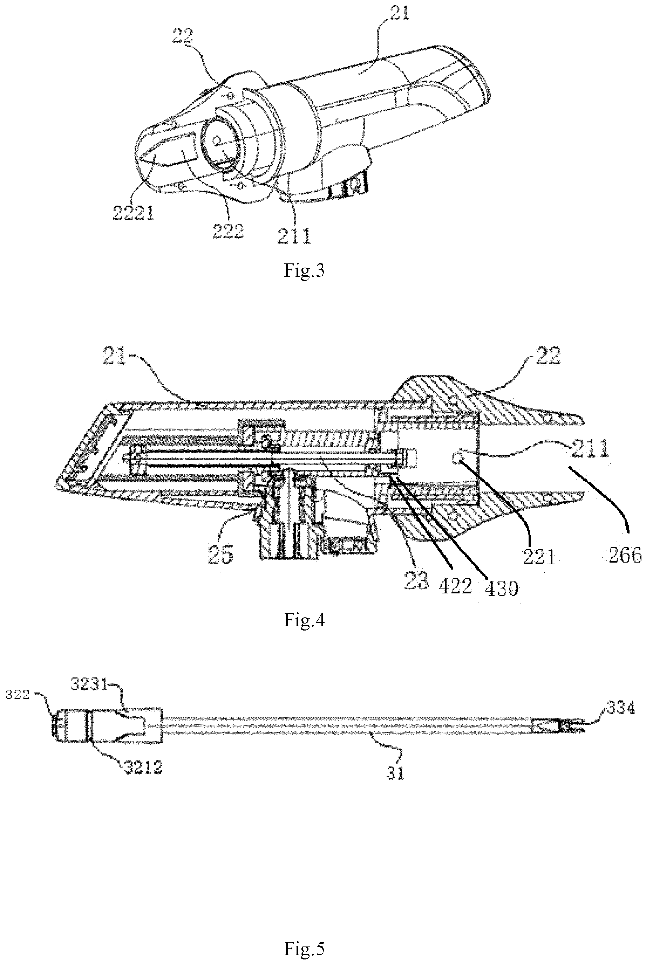

Fig. 3 is a schematic structural diagram of a working component and a rotary head provided in accordance with an embodiment of the present disclosure. Fig. 4 is a schematic cross-sectional view of Fig. 3.

As shown in Figs. 2 to 4, the working component 2 includes a first housing 21. An opening 211 is located on the first housing 21 of the working component 2 and is provided at the distal end of the first housing 21. The adapter further includes a rotary head 22, the rotary head 22 is sleeved onto the opening 211 and is configured to be rotatable relative to the first housing 21. As shown in Figs. 3 and 4, the rotary head 22 includes a first through hole 266 and a first guide member 222 disposed in the first through hole 266.

As shown in Fig. 2, the shaft assembly 3 includes a firing sleeve 31 and a coupling component 32. The coupling component 32 is provided at an end of the firing sleeve 31, and the coupling component 32 is inserted into the opening 211 of the first housing 21 after passing through the first through hole 266 of the rotary head 22.

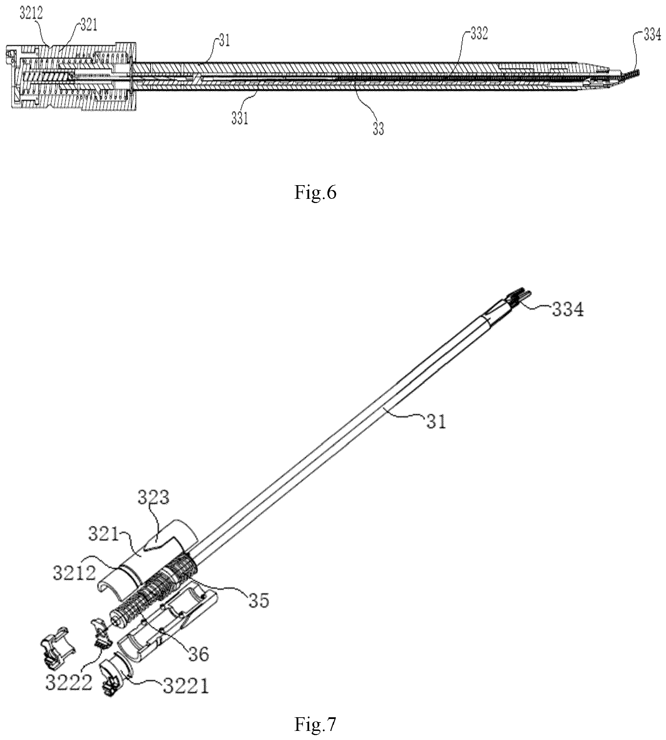

Fig. 7 is an exploded schematic view of a shaft assembly provided in accordance with an embodiment of the present disclosure. As shown in Fig. 7, the coupling component 32 includes a coupling casing 321 and a second guide member 323 provided on the coupling casing 321. The first guide member 222 and the second guide member 323 are engaged with each other in a snap-fit manner to maintain the rotary head 22 and the shaft assembly 3 in a locked position.

In the present embodiment, by engaging the first guide member 222 in the rotary head 22 with the second guide member 323 in the coupling component 32 of the shaft assembly 3, the shaft assembly 3 is rapidly coupled with the adapter (or the working component 2), thereby achieving a simple, fast, and convenient hemostatic operation.

For example, as shown in Figs. 1 to 4, while the rotary head 22 rotates, the coupling casing 321 also rotates around a central axis B of the rotary head 22 (or the shaft assembly 3). The coupling component 32 is inserted into the rotary head 22 along a direction parallel to the central axis B. In this way, it is convenient to adjust a coupling angle of the first guide member 222 and the second guide member 323, and to further realize quick abutting.

In the present embodiment, the design of the first guide member 222 and the second guide member 323 is to achieve quick, convenient and highly efficient abutting. For example, as shown in Figs. 2 and 3, the first guide member 222 has a first wedge portion tapered in a first direction (the +A direction shown in the figure) parallel to the center axis B, and the second guide member 323 has a second wedge portion tapered in a second direction (the -A direction shown in the figure) parallel to the center axis B, and the first direction is opposite to the second direction. Thus, while the first guide member 222 and the second guide member 323 are coupled with each other, each of the first wedge portion and the second wedge portion plays a role of guiding, because they both have a shape tapered from wide to narrow, thereby facilitating quick coupling between the first guide member 222 and the second guide member 323.

Further, as shown in Figs. 2 and 3, the first guide member 222 includes at least two first protrusions 2221 spaced apart from each other, and the second guide member 323 includes at least a second protrusion 3231 engaged between the at least two first protrusions 2221 in a snap-fit manner. The first protrusion 2221 and the second protrusion 3231 have an arrow-shaped structure. An arrow portion of the first protrusion 2221 is arranged opposite to an arrow portion of the second protrusion 3231 in preparation for insertion; after the insertion is completed, the arrow portions of the first protrusion 2221 and the second protrusion 3231 are arranged to be staggered and facing away from each other.

In other embodiments, depending on actual situations, the first protrusion 2221 and the second protrusion 3231 may have other shapes which are not particularly limited herein. In the present embodiment, the numbers of the first protrusion 2221 and the second protrusion 3231 are not particularly limited, which also depends on actual situations.

For example, as shown in Fig. 4, a driving rod 23 and a first communication assembly 422 are further provided in the first housing 21. Fig. 5 is a schematic structural diagram of a shaft assembly provided in accordance with an embodiment of the present disclosure. Fig. 6 is a schematic cross-sectional view of Fig. 5. As shown in Figs. 5 and 6, a second communication assembly 322 is provided at the proximal end of the coupling casing 321, and is connected with the first communication assembly 422 in a plugged-in manner so as to communicate with each other. When the shaft assembly 3 is inserted into the rotary head 22, the first guide member 222 and the second guide member 323 are cooperated with each other so that the first communication assembly 422 and the second communication assembly 322 are connected with each other in a plugged-in manner, thereby realizing signal transmission between the first communication assembly and the second communication assembly.

In order to facilitate the plugged-in connection between the first communication assembly 422 and the second communication assembly 322, as shown in Fig. 7, the second communication assembly 322 includes a communication casing 3221 mounted on the coupling casing 321 and an electrical terminal 3222 disposed in the communication casing 3221. The communication casing 3221 is rotatable relative to the coupling casing 321 so as to facilitate the plugged-in connection between the first communication assembly 422 and the second communication assembly 322.

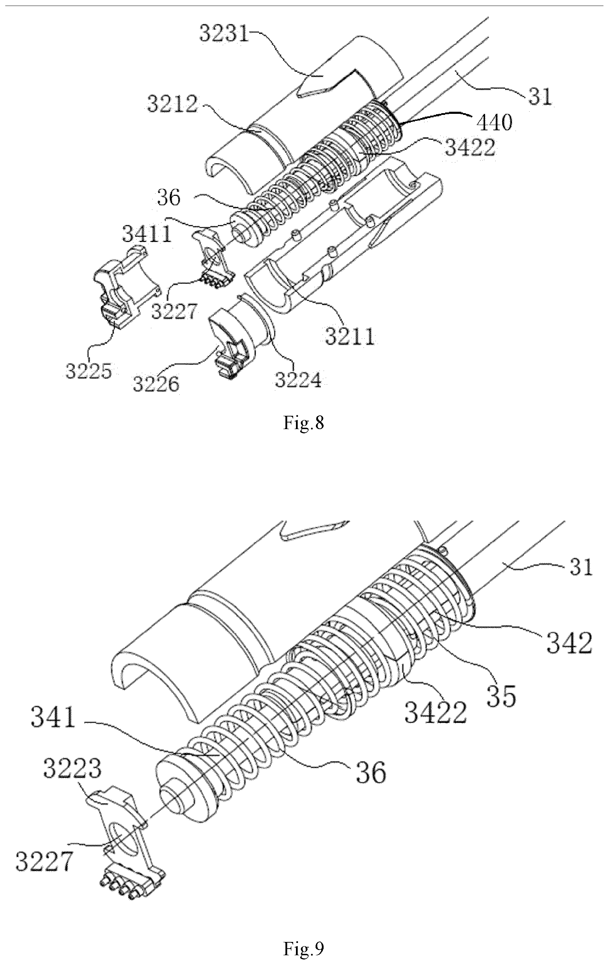

Fig. 8 is an exploded schematic view of a coupling component and a shaft assembly provided in accordance with an embodiment of the present disclosure. Further, as shown in Fig. 8, for example, the communication casing 3221 is provided with a rectangular opening 3225 to expose the electrical terminal 3222. As shown in Fig. 4, the first communication assembly 422 includes an abutting interface 430, which is connected with the rectangular opening 3225 in a plugged-in manner and aligned with the rectangular opening 3225 during the connection.

For example, the communication casing 3221 has a column-shaped structure. For example, a cross section of the column-shaped structure includes but is not limited to a circular shape, a rectangular shape, a triangular shape or other shapes. The embodiment of the present disclosure is described with reference to the case of cylindrical structure by way of example.

As shown in Fig. 8, an annular protrusion 3224 is formed on an outer cylindrical surface of the communication casing 3221, and the coupling casing 321 is provided with a first groove 3211 engaged with the protrusion 3224, whereby the second communication assembly 322 is rotatable relative to the coupling casing 321.

Further, a first locking member is provided on the coupling casing, a second locking member is provided within the rotary head, and the first locking member and the second locking member are locked with each other so as to keep the positions of the coupling casing and the rotary head fixed relative to each other.

For example, as shown in Fig. 8, a second groove 3212 is provided on the coupling casing 321. As shown in Fig. 4, a protrusion 221 (e.g., a sphere-shaped) is provided inside the rotary head 22. The second groove 3212 and the protrusion 221 are engaged with each other in a snap-fit manner to be locked with each other, so that the positions of the coupling casing 321 and the rotary head 22 are further kept fixed relative to each other, and the first guide member 222 and the second guide member 323 are not prone to be moved relative to each other.

For example, the coupling casing 321 also has a column-shaped structure. The coupling casing 321 thereon is provided with a second groove 3212, and the rotary head 22 therein is provided with a protrusion 221 which is engaged with the second groove 3212 in a snap-fit manner. The second groove 3212 and the protrusion 221 are locked with each other to keep the positions of the coupling casing 321 and the rotary head 22 fixed relative to each other.

In the above embodiment, the process for plugging the shaft assembly 3 into the rotary head 22 includes: rotating the communication casing 3221 relative to the coupling casing 321 to adjust the position of the second communication assembly 322, so that the positions of the second communication assembly 322 and the first communication assembly 422 correspond to each other, at the same time, the second protrusion 3231 substantially corresponds to the gap between the two first protrusions 2221. While the shaft assembly 3 is inserted into the rotary head 22, the position of the shaft assembly 3 is slightly adjusted so that the second protrusion 3231 is engaged with the first protrusions 2221 and the rectangular opening 3225 is completely coupled with the abutting interface 430; in this case, the second groove 3212 is engaged with the protrusion 221 in a snap-fit manner, and the second communication assembly 322 is connected with the first communication assembly 422 in a plugged-in manner. After the plugged-in connection between the shaft assembly 3 and the rotary head 22 is completed, the angle of the shaft assembly 3 is allowed to be adjusted by rotating the rotary head 22. Because the second communication assembly 322 and the first communication assembly are in coupling state and kept fixed, the coupling casing 321 is allowed to be rotated relative to the second communication assembly 322 so as to adjust the angle of the shaft assembly 3 within a range of 360 degrees.

For example, as shown in Figs. 2 and 4, the working component 2 further includes a driving rod 23 provided in the first housing 21, and the driving rod 23 is in a reciprocating motion along a direction parallel to the central axis B of the rotary head 22.

For example, as shown in Fig. 6, the shaft assembly 3 further includes a clip-cartridge assembly 33, which is located in the firing sleeve 31 and is configured to provide and deliver a ligating clip. The clip-cartridge assembly 33 includes a clip tray for carrying the ligating clip 332 and a clip-pushing piece 331 for pushing the ligating clip 332 to move forward. For example, the surgical apparatus further includes a clip jaw 334 connected to the clip tray, and the clip-pushing piece 331 delivers the ligating clip 332 to the clip jaw 334.

Fig. 9 is a partially exploded schematic view of a coupling component provided in accordance with an embodiment of the present disclosure. For example, as shown in Fig. 9, the shaft assembly 3 further includes a rod assembly 34 connected to the driving rod 23. The rod assembly 34 includes: a pushing rod 341 connected to the clip-cartridge assembly 33; and a firing rod 342 connected to the firing sleeve 31. The firing rod 342 is provided with a hollow portion, in which the pushing rod 341 is disposed and slidable with respect to the firing rod 342; the pushing rod 341 moves forward (i.e., in the +A direction shown in Fig. 2) so that the clip-cartridge assembly 33 delivers the ligating clip to the clip jaw 334 (simply referred to as "deliver the clip"); the pushing rod 341 continues to move forward against the firing rod 342 so as to drive the firing sleeve 31 to perform a firing action.

For example, as shown in Fig. 9, the second communication assembly 322 further includes a supporting member 3223, which is located in the communication casing 3221 and configured to support the electrical terminal 3222. For example, the supporting member 3223 may be a circuit board. The supporting member 3223 is provided with a second through hole 3227, and the supporting member 3223 is connected with the pushing rod 341 through the second through hole 3227.

For example, as shown in Fig. 8, the communication casing 3221 is further provided with a third through hole 3226, the position of the third through hole 3226 corresponds to the position of the second through hole 3227. The driving rod 23 sequentially passes through the third through hole 3226 and the second through hole 3227 and then is coupled with the pushing rod 341, thereby pushing the clip-pushing piece 331 to deliver the ligating clip and driving the firing sleeve 31 to perform a firing action so as to close the clip jaw 334.

For example, as shown in Figs. 8 and 9, the firing sleeve 31 has a flange 440, and the firing rod 342 is provided with a bump 3422 and a first restoration member 35; the first restoration member 35 is disposed between the bump 3422 and the flange 440 to restore the firing rod 342 to its original position.

For example, as shown in Figs. 8 and 9, one end of the pushing rod 341 is provided with a stop block 3411, a second restoration member 36 is provided between the stop block 3411 and the bump 3422 and is configured to restore the pushing rod 341 to its original position. Further, a diameter of the stop block 3411 is greater than or equal to a diameter of the firing rod 342 so as to prevent the pushing rod 341 from sliding into the firing rod 342. The first restoration member 35 and the second restoration member 36 may be elastic members, such as springs.

In the embodiment of the present disclosure, the rotary head 22, the coupling casing 321 and the communication casing 3221 may be separately disposed or integrally disposed depending on actual situations, which is not particularly limited herein.

For example, as shown in Fig. 2, the handheld component 1 is an electric handheld component and includes: a second housing 13 having a receiving cavity, an electric motor 14 disposed in the receiving cavity, a control assembly electrically connected to the electric motor 14, as well as a first switch 11 and a second switch 12 which are both disposed on the second housing 13 and coupled to the control assembly.

For example, as shown in Fig. 4, the working component 2 is further provided with a transmission mechanism 25, the transmission mechanism 25 is configured to convert a radial force of the electric motor 14 into an axial force, and the driving rod 23 is connected to the transmission mechanism 25. The first switch 11 serves to drive the driving rod 23 to move forward (e.g., in the +A direction shown in Fig. 2), and the second switch 12 serves to drive the driving rod 23 to move backward (e.g., in the -A direction shown in Fig. 2). It is to be understood that the driving assembly, the control assembly, the first switch 11 and the second switch 12 may also be provided in the working component 2 depending on actual situations, which is not particularly limited in the embodiments of the present disclosure.

The operational process of the surgical apparatus according to the embodiments of the present disclosure as described above is as below: the electric motor 14 drives the driving rod 23 to move forward, and the driving rod 23 serves to push the pushing rod 341 to move forward, so as to push the ligating clip 332 to move to the clip jaw 334, during this period of time, the second restoration member 36 is gradually compressed. Next, the pushing rod 341 continuously moves forward to abut against the firing rod 342 and drives the firing rod 342 to move forward, so that the first restoration member 35 pushes the firing sleeve 31 to move forward. Until the ligating clip 332 is delivered to the clip jaw 334, the firing sleeve 31 simultaneously closes the clip jaw 334 to fire the ligating clip (simply referred to as "firing"), at this time, the first restoration member 35 is in a compressed state. The electric motor 14 drives the driving rod 23 to move backward; while the external force on the first restoration member 35 is removed, the first restoration member 35 in the compressed state drives the firing rod 342 to restore to its original position under the action of an elastic force of the first restoration member 35 itself; while the external force on the second restoration member 36 is removed, the second restoration member 36 drives the pushing rod 341 to restore to its original position under the action of an elastic force of the second restoration member 36 itself.

Another embodiment of the present disclosure further provides a surgical apparatus, which not only realizes the rapid coupling between the shaft assembly and the adapter, but also solves the problems of complex clip-application process and poor surgical efficiency in the conventional manual clip-delivery mode.

The surgical apparatus provided by another embodiment of the present disclosure includes an adapter and a shaft assembly. The adapter includes a casing having an opening provided at a distal end of the casing. The adapter further includes a rotary head sleeved onto the opening of the casing and configured to be rotatable relative to the casing. The rotary head includes a first through hole and a first guide member provided in the first through hole. The shaft assembly is detachably connected to the adapter and includes a firing sleeve and a coupling component. The coupling component is disposed at an end of the firing sleeve and is configured to insert into the opening of the casing after passing through the first through hole of the rotary head. The coupling component includes a coupling casing and a second guide member disposed on the coupling casing. The first guide member and the second guide member are engaged with each other in a snap-fit manner to maintain the rotary head and the shaft assembly in a locked position. Further, the adapter further includes a working component and a handheld component detachably connected to the working component, and the shaft assembly is inserted into the working component. The shaft assembly includes: a clip-cartridge assembly located in the firing sleeve and configured to provide a ligating clip and to deliver the ligating clip; and a clip jaw. The surgical apparatus further includes an attitude sensing assembly and a control assembly electrically connected with the attitude sensing assembly. The attitude sensing assembly is configured to acquire attitude data of the working component and send the attitude data to the control assembly. The control assembly is configured to determine whether to activate an automatic clip-delivery mode based on the attitude data and determine whether a ligating clip is placed on the clip jaw. In the case that it is determined to activate the automatic clip-delivery mode and that no ligating clip is placed on the clip jaw, the clip-cartridge assembly automatically delivers the ligating clip to the clip jaw.

The surgical apparatus capable of achieving an automatic clip delivery according to an embodiment of the present disclosure is described in further detail below with reference to the accompanying drawings.

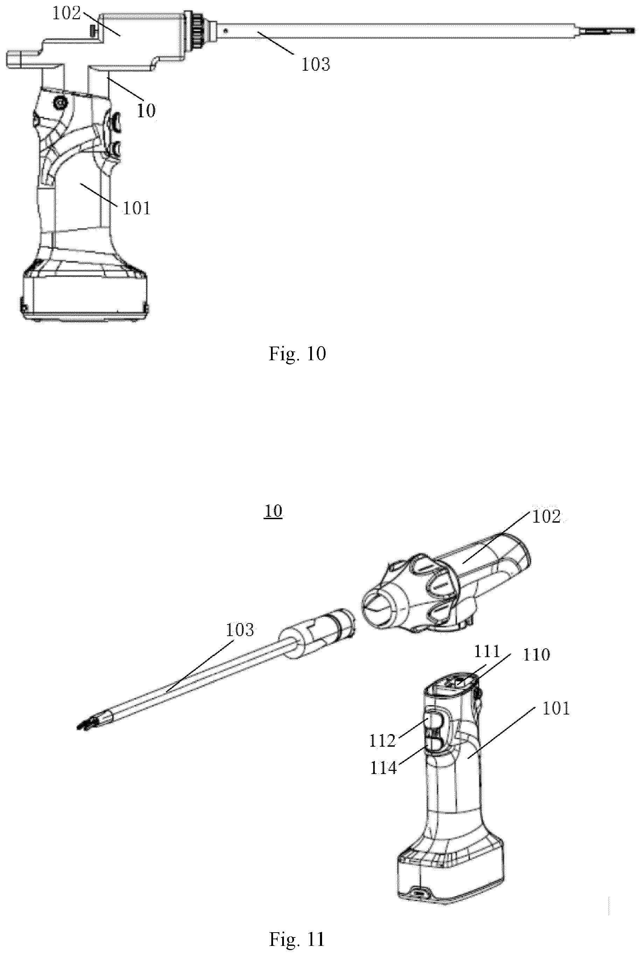

Fig. 10 is a schematic structural diagram of a surgical apparatus provided in accordance with another embodiment of the present disclosure. Fig. 11 is an exploded schematic view of a surgical apparatus provided in accordance with another embodiment of the present disclosure.

As shown in Figs. 10 and 11, the surgical apparatus 10 includes at least an adapter and a shaft assembly 103. For example, the adapter includes a handheld component 101, a working component 102 connected to the handheld component 101, and a shaft assembly 103 connected to the working component 102. Optionally, the handheld component 101 is detachably connected with the working component 102; the work member 102 is detachably connected with the shaft assembly 103.

It should be noted that, in the present embodiment, only the differences between Fig. 10 and Fig. 1 are explained, and the similarities are not described in detail. For example, the arrangement of the first guide member and the second guide member in Fig. 10 is the same as that in Fig. 1, which is repeatedly described herein.

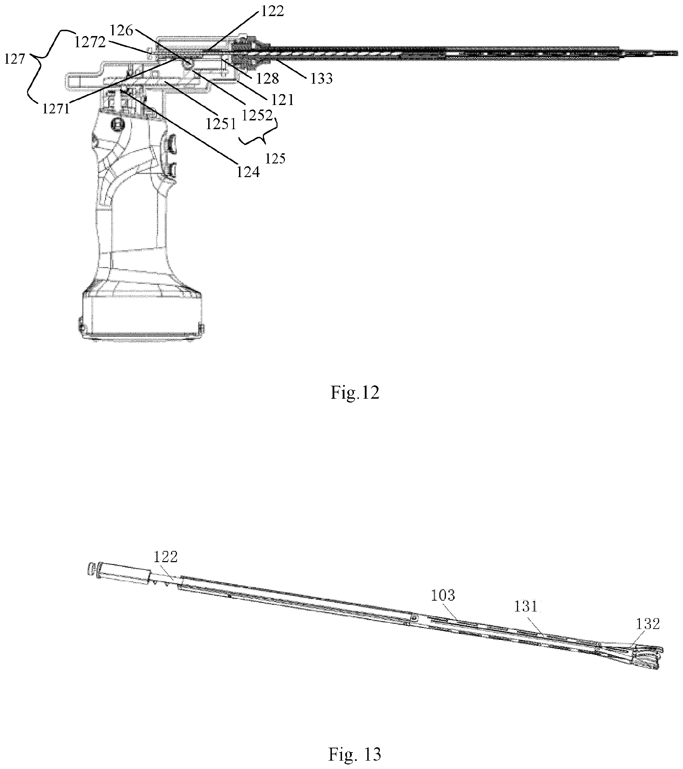

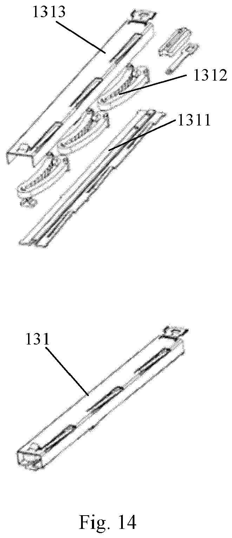

For example, as shown in Figs. 10 and 11, the shaft assembly 103 is inserted into the working component 102. As shown in Figs. 13 and 14, the shaft assembly 103 includes a clip-cartridge assembly 131, which is located in the firing sleeve (reference may be made to the positional relationship of the clip-cartridge assembly 33 and the firing sleeve 31 in the embodiment of Fig. 6) and configured to provide a ligating clip 1312 and deliver the ligating clip 1312. The shaft assembly 103 further includes a clip jaw 132.

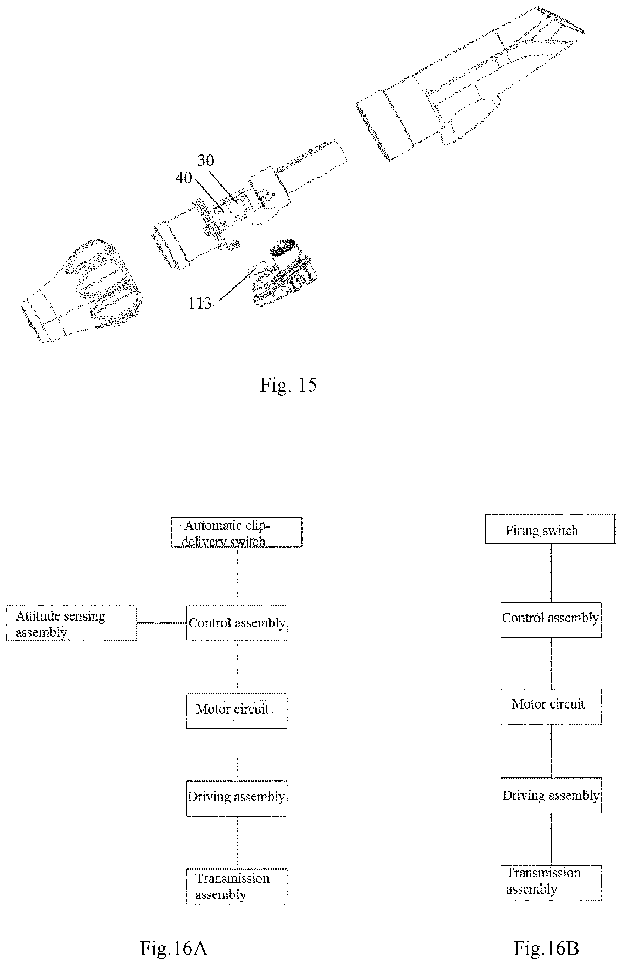

For example, as shown in Fig. 15, the surgical apparatus 10 further includes an attitude sensing assembly 30 and a control assembly 40 communicatively connected to the attitude sensing assembly 30. The attitude sensing assembly 30 is configured to acquire attitude data of the working component and send the attitude data to the control assembly 40. The control assembly 40 is configured to determine whether to activate an automatic clip-delivery mode based on the attitude data and determine whether a ligating clip is placed on the clip jaw. In the case that it is determined to activate the automatic clip-delivery mode and that no ligating clip 1312 is placed on the clip jaw 132, the clip-cartridge assembly 131 automatically delivers the ligating clip 1312 to the clip jaw 132. In the present embodiment, by providing the attitude sensing assembly 30 and the control assembly 40 communicatively connected to the attitude sensing assembly 30, the problems that the medical practitioner needs to manually press a clip-delivery switch to deliver the ligating clip to the clip jaw 132 every time during a surgery can be avoided, and thus the clip-application process is simplified.

Various components and working principle(s) of the surgical apparatus of the embodiments of the present disclosure are briefly described below for better understanding. It should be noted that the surgical apparatus may also be referred to as a clip applicator, a surgical device, and the like.

For example, as shown in Fig. 11, the handheld component 101 includes a second housing 110 and a driving assembly 111 disposed in the second housing 110. Fig. 15 is an exploded schematic view of a working component provided in accordance with another embodiment of the present disclosure. As shown in Figs. 11 and 15, the handheld component 101 is further provided with a firing switch 112 configured to control the driving assembly 111, and a motor circuit 113 connected to the firing switch 112; and the motor circuit 113 is further connected to the driving assembly 111. As shown in Fig. 16, the motor circuit 113 is configured to control the movement of the driving assembly 111. For example, the driving assembly 111 includes a driving motor. The driving motor has an output shaft thereon.

For example, the automatic delivery of the ligating clip 1312 from the clip-cartridge assembly 131 to the clip jaw 132 is achieved by: controlling the driving assembly 111 to move, so that the driving assembly drives the pushing rod 122 to push the clip-cartridge assembly 131 to automatically deliver the ligating clip 1312 to the clip jaw 132.

Fig. 12 is a schematic cross-sectional view of a working component provided in accordance with another embodiment of the present disclosure. For example, as shown in Fig. 12, the working component 102 includes: a first housing 121; a transmission assembly provided in the first housing 121 and connected to the driving assembly 111; and a pushing rod 122 connected to the transmission assembly.

For example, the transmission assembly includes: a transmission shaft connected to the driving assembly 111 (e.g., an output shaft of a driving motor), a bevel gear 124 connected to the transmission shaft, a rack assembly 125 engaged with the bevel gear 124, a pinion gear 126 engaged with the rack assembly 125, and a pushing assembly 127 connected to the pinion gear 126. The rack assembly 125 includes a first rack 1251 connected to the bevel gear 124 and a second rack 1252 connected to the first rack 1251; the second rack 1252 is further connected to a firing forcing member 128.

For example, as shown in Fig. 12, the pushing assembly 127 is configured to push the pushing rod 122 to move under the drive of the pinion gear 126. In one example, the pushing assembly 127 includes a pushing block 1271 engaged with the pinion gear 126, and a pushing member 1272 connected to the pushing block 1271; the pushing member 1272 drives the pushing rod 122 to move.

Fig. 13 is a schematic structural diagram of a shaft assembly provided in accordance with another embodiment of the present disclosure. For example, as shown in Fig. 13, the clip-cartridge assembly 131 includes at least one ligating clip 1312. The clip-cartridge assembly 131 is connected to the pushing rod 122, and a reciprocating motion of the pushing rod 122 drives the clip-cartridge assembly 131 to deliver the ligating clip.

Fig. 14 is a schematic structural diagram of a clip-cartridge assembly provided in accordance with another embodiment of the present disclosure. Optionally, as shown in Fig. 14, the clip-cartridge assembly 131 includes a clip-cartridge holder 1311, a ligating clip 1312 placed in the clip-cartridge holder 1311, and a clip-pushing piece 1313 provided to cover the clip-cartridge holder 1311, the clip-pushing piece 1313 is configured to push the ligating clip 1312 to move. When the clip jaw 132 is closed, the pushing rod 122 drives the clip-pushing piece 1313 to push the ligating clip 1312 to move towards the clip jaw 132. The clip-pushing piece 1313 is provided with a plurality of ratchets facing towards the clip jaw 132, and these ratchets are abutted against the ligating clip 1312 so as to push the ligating clip 1312 to move towards the clip jaw 132 under the push of the clip-pushing piece 1313.

For example, as shown in Fig. 12, the shaft assembly 103 further includes a firing member 133. One end of the firing member 133 is connected to the firing forcing member 128, and the other end of the firing member 133 is connected to the clip jaw 132. The movement of the firing forcing member 128 drives the firing member 133 to control the opening and closing of the clip jaw 132.

Optionally, the firing member 133 is a firing sleeve that wraps around the clip-cartridge assembly 131. In other embodiments, the firing member 133 may also be implemented as a pulling rod connected to the clip jaw 132, and the other end of the pulling rod is connected to the firing forcing member 128 by a connector.

During the usage, in the case that the clip-cartridge assembly needs to deliver the ligating clip, the driving assembly 111 rotates in a certain direction (e.g., a third direction) and the output shaft drives the bevel gear 124 to rotate; the bevel gear 124 drives the first rack 1251 to move backward and further drives the second rack 1252 to move backward. The second rack 1252 moves backward so as to be engaged with the pinion gear 126. At this time, the second rack 1252 drives the pinion gear 126 to rotate clockwise. The pinion gear 126 rotates clockwise so as to push the pushing block 1271 to move forward. The pushing block 1271 moves forward so as to drive the pushing member 1272 to push the pushing rod 122 to move. An axial movement of the pushing rod 122 towards the clip jaw 132 drives the clip-cartridge assembly 131 to deliver the ligating clip.

In the case that the clip-cartridge assembly needs to withdraw, an axial movement of the pushing rod 122 towards the handheld component 101 is in the same manner as the axial movement of the pushing rod 122 towards the clip jaw 132 except that, at this time, the driving assembly 111 rotates in a fourth direction (a direction opposite to the third direction) and drives other transmission assemblies to move in an opposite direction, so that the pushing rod 122 moves axially towards the handheld component 101 and drives the clip-cartridge assembly 131 to withdraw.

Next, in the case that the clip jaw needs to close for ligation, the driving assembly 111 rotates in the fourth direction and the output shaft drives the bevel gear 124 to rotate; the bevel gear 124 drives the first rack 1251 to move forward and further drives the second rack 1252 to move forward. The forward movement of the second rack 1252 drives the firing forcing member 128 to move forward, and the forward movement of the firing forcing member 128 drives the firing member 133 to move forward relative to the clip-cartridge assembly 131, so that the clip jaw 132 is closed and the ligation is completed.

In the case that the clip jaw needs to open, the firing forcing member 128 moves towards the handheld component 101 in the same manner as the firing forcing member 128 moving towards the clip jaw 132 except that, in this situation, the driving assembly 111 rotates in the third direction to drive other transmission assemblies to move in an opposite direction, so that the firing forcing member 128 moves axially towards the handheld component 101 and drives the firing member 133 to withdraw, thereby opening the clip jaw 132.

In the embodiments of the present disclosure, the attitude sensing assembly includes an acceleration sensor, and the attitude data includes acceleration data of the surgical apparatus. For example, an acceleration may be generated in the case that the medical practitioner picks up or moves the surgical apparatus. The acceleration sensor (or gyroscope) is a kind of sensor that measures the acceleration and usually consists of a mass block, a damper, an elastic element, a sensitive element and an adaptive circuit, etc. During the accelerated movement of the sensor, an acceleration value may be obtained by measuring an inertial force subjected to the mass block based on Newton's second law. Optionally, a voltage output by the acceleration sensor is proportional to the acceleration. For example, the voltage of 2.5 V corresponds to an acceleration of 0 g, and the voltage of 2.6 V corresponds to an acceleration of 0.5 g (approximately equal to 1.9 m/s²). The control assembly includes a controller such as a mainboard (e.g., a circuit board).

By way of example, the acceleration sensor may be a three-axis acceleration sensor, and may also be a six-axis acceleration sensor, a nine-axis acceleration sensor, and the like. The present embodiment is not intended to limit the particular type of the acceleration sensor.

In one example, the attitude sensing assembly 30 and the control assembly 40 are disposed in the working component 102, as shown in Fig. 15. In other embodiments, the attitude sensing assembly 30 and the control assembly 40 may also be disposed in the handheld component 101; alternatively, the attitude sensing assembly 30 is provided in the handheld component 101, and the control assembly 40 is provided in the working component 102; alternatively, the attitude sensing assembly 30 is provided in the working component 102 and the control assembly 40 is provided in the handheld component 101. The positions of the attitude sensing assembly 30 and the control assembly 40 are not particularly limited in the present embodiment.

In the embodiments of the present disclosure, the control assembly 40 may determine whether to activate an automatic clip-delivery mode in at least two ways. The first way is that, the control assembly determines whether to activate the automatic clip-delivery mode according to whether the attitude data satisfies a starting process and/or an ending process of a surgery; and the other way is that, the control assembly determines whether to activate the automatic clip-delivery mode according to whether an automatic clip-delivery signal is received and whether the attitude data satisfies a surgery ending process. Different scenarios suitable for these two ways will be explained below.

### The First Way

The first scenario: the medical practitioner picks up a surgical apparatus 10 and starts a surgery by using the surgical apparatus 10. In this situation, the number of the ligating clip having been delivered to the clip jaw 132 by the surgical apparatus 10 is zero.

In this case, the control assembly 40 is further configured to: determine to activate an automatic clip-delivery mode if a variation amount of the acceleration data within a first preset time period is matched with a first template variation rule, in the case that the number of the ligating clip having been delivered to the clip jaw is zero.

For example, the first template variation rule is established based on a variation amount of acceleration data during the starting process of the surgery performed by using the surgical apparatus 10.

Because the shaft assembly 103 of the surgical apparatus 10 is to be inserted into the human body while the medical practitioner starts to use the surgical apparatus 10, the medical practitioner moves the surgical apparatus 10 along the central axis of the shaft assembly 103 towards the clip jaw 132. Based on this principle, the first template variation rule is that: the variation amount of displacement, along the central axis of the shaft assembly 103 and towards the clip jaw 132, within the first preset time period is greater than a first preset value.

For example, the first preset time period is 5 seconds (5 s), 10 seconds (10 s), and the like. In this embodiment, the value of the first preset time period is not limited. The first preset value may be 5 cm, 3 cm, and the like. In this embodiment, the first preset value is not limited.

The second scenario: the medical practitioner is operating a surgery by using the surgical apparatus 10. In this situation, the number of the ligating clip(s) having been delivered to the clip jaw 132 by the surgical apparatus 10 is greater than zero.

In this case, the control assembly 40 is further configured to: determine to activate the automatic clip-delivery mode if the variation amount of acceleration data within a second preset time period is not matched with a second template variation rule, in the case that the number of the ligating clip(s) having been delivered to the clip jaw 132 is greater than zero. For example, the second template variation rule is established based on the variation amount of acceleration data in the ending process of the surgery operated by using the surgical apparatus 10. If the variation amount of the acceleration data within the second preset time period is matched with the second template variation rule, the automatic clip-delivery mode is not activated.

During the usage of the surgical apparatus 10, if the ligation position needs to be changed, it has to move the surgical apparatus 10 and the displacement amount is small. However, if it needs to end the surgery, the shaft assembly 103 of the surgical apparatus 10 has to be pulled out of the human body; in this situation, the medical practitioner controls the surgical apparatus 10 to move along the central axis of the shaft assembly 103 towards the handheld component 101, thus, the displacement amount is greater larger than the displacement amount of the medical apparatus moved in the human body. Based on this principle, the second template variation rule is that, the variation amount of the displacement along the central axis of the shaft assembly 103 towards the handheld component 101 within the second preset time period is greater than a second preset value. In this case, if the variation amount of the acceleration data within the second preset time period is not matched with the second template variation rule, it indicates that the surgical apparatus 10 has not been pulled out of the human body, and the automatic clip-delivery needs to be continued.

For example, the second preset time period may be 3 s, 5 s, 10 s, etc. In this embodiment, the value of the second preset time period is not limited. The second preset value may be 5 cm, 3 cm, and the like. In this embodiment, the value of the second preset value is not limited. Optionally, the second preset value is equal to the first preset value.

### The Second Way:

Fig. 16A is a block diagram of an electrical connection relationship between an automatic clip-delivery switch and other components in a surgical apparatus provided in accordance with another embodiment of the present disclosure.

As shown in Fig. 16A, for example, the handheld component 101 further includes an automatic clip-delivery switch connected to the control assembly 40, and the automatic clip-delivery switch is triggered. An automatic clip-delivery signal is sent to the control assembly 40 when the automatic clip-delivery switch is triggered. In the embodiment of the present disclosure, "the automatic clip-delivery switch is triggered" means that the automatic clip-delivery switch sends an automatic clip-delivery signal, but does not mean that an automatic clip-delivery action is performed. The automatic clip-delivery action is performed only in the case that the control assembly determines to activate the automatic clip-delivery mode.

The control assembly 40 is further configured to determine whether to activate the automatic clip-delivery mode upon receiving the automatic clip-delivery signal. For example, as shown in Fig. 16, the attitude sensing assembly acquires the acceleration data of the surgical apparatus (e.g., acquiring the data of the working component or the handheld component) and determines to activate the automatic clip-delivery mode if a variation amount of the acceleration data within the second preset time period is not matched with the second template variation rule.

Next, in the case that it is determined to activate the automatic clip-delivery mode and that the ligating clip is not placed on the clip jaw 132, the control assembly 40 sends an automatic clip-delivery control signal to the motor circuit 113. The motor circuit 113 is configured to receive the automatic clip-delivery control signal from the control assembly 40 and drives the driving assembly 111 to move. The driving assembly 111 drives the transmission assembly to move, and the pushing rod 112 performs an axial movement under the drive of the transmission assembly so as to push the clip-cartridge assembly 131 to automatically deliver the ligating clip 1312 to the clip jaw 132, thereby completing the automatic clip-delivery action.

In the embodiments of the present disclosure, after the automatic clip-delivery action is completed, the medical practitioner manually operates the firing switch to fire the ligating clip to the target tissue of the human body.

Fig. 16B is a block diagram of an electrical connection relationship between a firing switch and other components in a surgical apparatus provided in accordance with another embodiment of the present disclosure.

As shown in Fig. 16B, for example, the firing switch 112 is connected to the control assembly 40. When the firing switch 112 is triggered, it sends a firing signal to the control assembly 40. In the present embodiment, "the firing switch is triggered" means that the firing switch sends out a firing signal. For example, the firing signal indicates to execute a firing action, and it may also mean that the ligating clip delivered last time has been fired.

In this situation, the control assembly 40 sends a firing control signal to the motor circuit 113. The motor circuit 113 controls the driving assembly 111 and the transmission assembly to move, so as to fire the ligating clip 113 on the clip jaw 132, thereby completing the firing action.

Optionally, the automatic clip-delivery switch and the firing switch 112 may be implemented as different switches; alternatively, the firing switch 112 may be implemented as a switch having an automatic clip-delivery trigger function, that is, the automatic clip-delivery switch and the firing switch 112 are provided as a single common switch.

For example, as shown in Fig. 11, the firing switch 112 of the present embodiment is a common switch having an automatic clip-delivery trigger function. For example, the firing switch 112 is connected to the control assembly 40; the firing switch 112 sends a firing signal to the control assembly 40 when the firing switch 112 is triggered; the control assembly 40 sends a firing control signal to the motor circuit 113, and the motor circuit 113 controls the driving assembly 111 to move so as to fire the ligating clip on the clip jaw 132.

The term "switch" as used in the embodiments of the present disclosure may be understood as a switching device that is switchable between at least two states.

For example, in the case that the firing switch and the automatic clip-delivery switch are provided as two switches independent of each other, both the firing switch and the automatic clip-delivery switch has a first state (e.g., an "on" state) and a second state (e.g., an "off' state). When the switch is in the first state, e.g., in a conducted state, a signal is sent. When the switch is in the second state, e.g., in a disconnected state, no signal is sent.

For example, in the case that the firing switch and the automatic clip-delivery switch are provided as the single common switch, the switch may be a switching device having at least three states. In such case, the switch has more than two states of "on" and "off'. For example, when the switch is in the first state, an automatic clip-delivery signal is sent out; when the switch is in the second state, a firing signal is sent out; and when the switch is in the third state, no signal is sent out.

For example, the switch having at least three states as described above includes but is not limited to a button and a slider. For example, in the case that the switch is provided as a button, the switching between the at least three states is achieved by setting the times of the pressing action(s) (e.g., pressing the button for one time, continuously pressing the button for two times, continuously pressing the button for three times, etc.). In the case that the switch is provided as a slider, the switching between different states is achieved by a pushing mode of the slider, and detailed description is omitted here.

For example, in the case that the medical practitioner uses the surgical apparatus 10 and presses the firing switch 112 for the first time, the surgical apparatus 10 does not deliver the ligating clip. In this situation, the firing switch is in the first state, only the automatic clip-delivery mode is triggered and the firing switch sends out the automatic clip-delivery signal. In the case that the firing switch 112 is pressed for the second time or the firing switch 112 is continuously pressed for two times, the surgical apparatus 10 performs the firing action. It should be understood that the switching between different states may be achieved by various manners, and that the switch may have various implementations, which are not particularly limited in the present disclosure.

It should be supplemented that, in this way, the control assembly 40 only receives an automatic clip-delivery signal for one time during a single surgery; in the subsequent process, the control assembly 40 only needs to determine whether the variation amount of the acceleration data within the second preset time period is matched with the second template variation rule (i.e., the case of the second scenario described above).

In the embodiment of the present disclosure, after the surgical apparatus 10 determines to activate the automatic clip-delivery mode, the surgical apparatus 10 does not need to perform the automatic clip-delivery action if a ligating clip is placed on the clip jaw 132. Thus, the surgical apparatus 10 further needs to determine whether a ligating clip is placed on the clip jaw 132.

After the medical practitioner presses the firing switch 112, the surgical apparatus 10 performs a firing action to fire the ligating clip on the clip jaw 132 to the target tissue of the human body. Thus, it means that, after the surgical apparatus 10 performs the firing action, there is no ligating clip placed on the clip jaw 132. At this time, if the automatic clip-delivery mode is activated, the surgical apparatus 10 performs the automatic clip-delivery action.

For example, in the case that the firing switch 112 is triggered, the firing switch 112 sends a firing signal to the control assembly 40. At this time, the control assembly 40 sends a firing control signal to the motor circuit 113. The motor circuit 113 controls the driving assembly 111 to move, so as to fire the ligating clip 113 on the clip jaw 132, thereby completing a firing action.

Further, upon the completion of the firing action, the surgical apparatus enters the previously described second scenario. In this situation, the control assembly 40 determines again whether to activate the automatic clip-delivery mode.

If it's determined to activate the automatic clip-delivery mode, the control assembly 40 sends an automatic clip-delivery control signal to the motor circuit 113. The motor circuit 113 receives the automatic clip-delivery control signal, and controls the driving assembly 111 to move, according to the automatic clip-delivery control signal, so that the transmission assembly drives the pushing rod 122 to push the clip-cartridge assembly 131 to automatically deliver the ligating clip to the clip jaw 132. In this way, the automatic clip-delivery is completed. In the present embodiment, because the clip-delivery is automatically performed after the firing action, it not only improves the efficiency and convenience of the surgery but also shortens the operational time of the surgery.

Optionally, as shown in Fig. 11, the handheld component 101 further includes another switch 114 connected to the control assembly 40. For example, the switch 114 has a function different from that of the firing switch 112, and the specific function of the switch 114 may be configured according to actual needs.

For example, the switch 114 has a manual clip-delivery function. After the medical practitioner presses the firing switch 112, the automatic clip-delivery mode is stopped if the switch 114 is further pressed. In this way, when the medical practitioner does not need the surgical apparatus to perform automatic clip-delivery, the automatic clip-delivery mode may be stopped by triggering the clip-delivery switch 114, then the ligating clip is allowed to be manually delivered. For example, the clip-delivery switch 114 is not arranged in the embodiment of the present disclosure.

In the surgical apparatus provided by the embodiments of the present disclosure described above, the problems of complex clip-application process and poor operational efficiency in the conventional manual clip-delivery mode can be solved by arranging the attitude sensing assembly and the control assembly. Whether the surgical apparatus is being used in the surgery is determined upon a current attitude of the surgical apparatus, and the ligating clip is capable of being automatically delivered to the clip jaw if it's determined that the surgical apparatus is being used in a surgery (i.e., the attitude data represents the attitude in a surgical procedure), thus the medical practitioner does not need to manually send the ligating clip during the surgery. Thus, the clip-application process can be simplified, and the efficiency of the surgery can be improved.

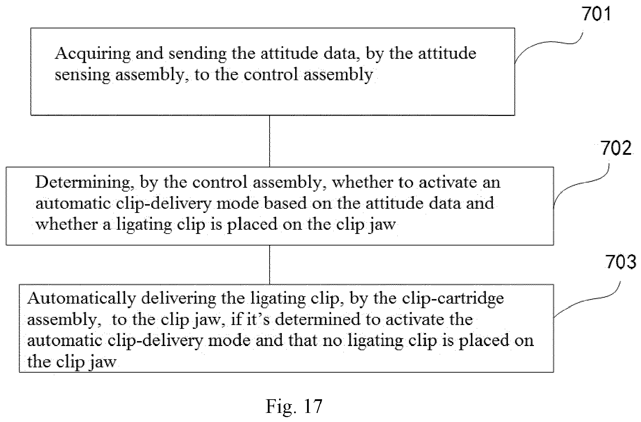

Fig. 17 is a flow chart of an automatic clip-delivery method provided in accordance with yet another embodiment of the present disclosure. The method may be performed by using the surgical apparatus including an attitude sensing assembly and a control assembly as described in the previous embodiments. For example, the automatic clip-delivery method includes steps as below.

Step S701, acquiring and sending the attitude data, by the attitude sensing assembly, to the control assembly. The attitude data serves for indicating the current attitude of the surgical apparatus.

Optionally, the attitude sensing assembly is an acceleration sensor; accordingly, the attitude data includes acceleration data of the surgical apparatus. As for the construction and operating principle of the acceleration sensor, reference can be made to the description in the previous embodiments, without repeatedly described here.

Step S702, determining, by the control assembly, whether to activate an automatic clip-delivery mode based on the attitude data and whether a ligating clip is placed on the clip jaw.

For example, the control assembly determines whether to activate the automatic clip-delivery mode based on the attitude data by at least the following two ways.

In the first way, the control assembly determines whether to activate an automatic clip-delivery mode based on whether the attitude data satisfies a starting process and/or an ending process of a surgery.

For example, in a first scenario, in the case that the number of the ligating clip having been delivered to the clip jaw is zero, it is determined to activate an automatic clip-delivery mode if a variation amount of the acceleration data within a first preset time period is matched with a first template variation rule.

For example, the first template variation rule is established based on a variation amount of acceleration data in a starting process of a surgery performed by using the surgical apparatus.

Optionally, the first template variation rule is that, a variation amount of displacement along a central axis of the shaft assembly towards the clip jaw within a first preset time period is greater than a first preset value.

In a second scenario, in the case that the number of the ligating clip(s) having been delivered to the clip jaw is greater than zero, it's determined to activate an automatic clip-delivery mode if a variation amount of the acceleration data within a second preset time period is not matched with a second template variation rule.

For example, the second template variation rule is established based on a variation amount of the acceleration data in an ending process of a surgery by using the surgical apparatus.

Optionally, the second template variation rule is that, a variation amount of displacement, along a central axis of the shaft assembly and towards the handheld component, within a second preset time period is greater than a second preset value.

In the second way, the control assembly determines whether to activate an automatic clip-delivery mode according to whether an automatic clip-delivery signal is received and whether the attitude data satisfies an ending process of a surgery.

For example, the handheld component further includes a motor circuit and an automatic clip-delivery switch. As for the specific settings and connection modes of the motor circuit, the automatic clip-delivery switch and the control assembly in this embodiment, reference can be made to the description in the previous embodiments, without repeatedly described here.

For example, the automatic clip-delivery switch sends an automatic clip-delivery signal to the control assembly when triggered. The control assembly is used for, upon receiving the automatic clip-delivery signal, determining to activate the automatic clip-delivery mode if the variation amount of the acceleration data within the second preset time period is not matched with the second template variation rule, and for sending an automatic clip-delivery control signal to a motor circuit. The motor circuit controls a movement of the driving assembly according to the automatic clip-delivery control signal, so that the transmission assembly drives the pushing rod to push the clip-cartridge assembly to automatically deliver the ligating clip to the clip jaw.

For example, the automatic clip-delivery signal is a signal sent by the automatic clip-delivery switch to the control assembly when the automatic clip-delivery switch is triggered. The automatic clip-delivery signal does not indicate an activation of the automatic clip-delivery mode, but merely brings the surgical apparatus into a ready state for automatic clip-delivery. Whether the automatic clip-delivery mode needs to be activated is determined by the control assembly.

In the embodiment of the present disclosure, the control assembly not only determines whether the automatic clip-delivery mode is activated, but also determines whether a ligating clip is placed on the clip jaw. Optionally, determining whether a ligating clip is placed on the clip jaw is before or after determining whether to activate the automatic clip-delivery mode; alternatively, at the same time of determining whether a ligating clip is placed on the clip jaw, determining whether to activate the automatic clip-delivery mode. The present embodiment does not limit the timing at which the control assembly determines whether a ligating clip is placed on the clip jaw.

For example, the handheld component further includes a firing switch connected to the control assembly. As for the specific settings and connection modes of the firing switch, reference can be made to the description of the previous embodiments, without repeatedly described herein. The firing switch sends a firing signal to the control assembly when triggered.

For example, determining whether a ligating clip is placed on the clip jaw includes:
determining that no ligating clip is placed on the clip jaw, if the clip-cartridge assembly is not controlled to deliver a ligating clip after receiving a firing signal.
determining that a ligating clip is placed on the clip jaw, if no firing signal is received. For example, the firing signal is sent by the firing switch to the control assembly when the firing switch is triggered.

For another example, determining whether a ligating clip is placed on the clip jaw includes:
determining that no ligating clip is placed on the clip jaw, if no automatic clip-delivery signal is received and the clip-cartridge assembly is not controlled to deliver a ligating clip after receiving a firing signal.
determining that a ligating clip is placed on the clip jaw, if no firing signal is received, or if both of a firing signal and an automatic clip-delivery signal are received. For example, the automatic clip-delivery signal is sent by the automatic clip-delivery switch to the control assembly when the automatic clip-delivery switch is triggered.

Step S703, controlling the driving assembly in the handheld component to move so that the clip-cartridge assembly automatically delivers the ligating clip to the clip jaw, if it's determined to activate the automatic clip-delivery mode and that no ligating clip is placed on the clip jaw.

For example, the control assembly sends an automatic clip-delivery control signal to the motor circuit, and the motor circuit controls the driving assembly to move according to the automatic clip-delivery control signal so that the transmission assembly drives the pushing rod to push the clip-cartridge assembly to automatically deliver the ligating clip to the clip jaw.

As for related components in the above-described method and details thereof, reference can be made to corresponding components in the previous embodiments, without repeatedly described in the present embodiment.

In summary, the automatic clip-delivery method provided by the present embodiment solves the problems of complex clip-application process and poor surgical efficiency in the conventional manual clip-delivery mode. Whether the surgical apparatus is being used in the surgery is determined upon a current attitude of the surgical apparatus, and the ligating clip is capable of being automatically delivered to the clip jaw if it's determined that the surgical apparatus is being used in a surgery (i.e., the attitude data represents the attitude in a surgical procedure), thus the medical practitioner does not need to manually send the ligating clip during the surgery. Thus, the clip-application process can be simplified, and the efficiency of the surgery can be improved.

In the present disclosure, the following statements should be noted:
(1) The accompanying drawings involve only the structure(s) in connection with the embodiment(s) of the present disclosure, and other structure(s) can be referred to general design(s).
(2) For the purpose of clarity, in accompanying drawings for illustrating the embodiment(s) of the present disclosure, the thickness and size of a layer or a region are not drawn according to an actual scale but are exaggerated or diminished. However, it should understood that, in the case an element such as a layer, a film, a region, a substrate or the like is referred to be "on" or "under" another element, the element may be "directly" disposed "on" or "under" another element, or there may be an intermediate element interposed therebetween.
(3) The embodiments of the present disclosure and the features therein can be combined with each other to obtain new embodiments in the absence of conflicts.

What is described above is related to the exemplary embodiments of the disclosure only and not limitative to the scope of the disclosure; the scope of the disclosure is defined by the accompanying claims.

## Claims

1. A surgical apparatus, comprising:
an adapter, comprising:
a casing, comprising an opening (211) provided at a distal end of the casing; and
a rotary head (22), sleeved onto the opening (211) of the casing and configured to be rotatable relative to the casing, the rotary head (22) comprising a first through hole (266) and a first guide member (222) disposed in the first through hole (266);
a shaft assembly (3), detachably connected to the adapter and comprising:
a firing sleeve (31); and
a coupling component (32), disposed at an end of the firing sleeve (31), the coupling component (32) being configured to insert into the opening (211) of the casing after passing through the first through hole (266) of the rotary head (22), the coupling component (32) comprising a coupling casing (321) and a second guide member (323) disposed on the coupling casing (321), **characterized in that**
the first guide member (222) comprises a first wedge portion tapered in a first direction (+A) parallel to a central axis (B) of the rotary head (22), and the second guide member (323) comprises a second wedge portion tapered in a second direction (-A) parallel to the central axis (B) of the rotary head (22), the first direction (+A) is opposite to the second direction (-A), and the first guide member (222) and the second guide member (323) are engaged with each other in a snap-fit manner to maintain both the rotary head (22) and the shaft assembly (3) in a locked position.

2. The surgical apparatus according to claim 1, wherein:
the rotary head (22) is configured to drive the coupling casing (321) to rotate around the central axis (B) of the rotary head (22), and the coupling component (32) is configured to insert into the rotary head (22) along a direction parallel to the central axis (B).

3. The surgical apparatus according to claim 1 or 2, wherein:
the first guide member (222) comprises at least two first protrusions (2221), and the at least two first protrusions (2221) are spaced apart from each other; and
the second guide member (323) is located on a surface of the coupling casing (321) and comprises a second protrusion (3231) which is engaged between the at least two first protrusions (2221) in a snap-fit manner.

4. The surgical apparatus according to any one of claims 1-3,
wherein the adapter comprises:
a working component (2) having a first housing (21), the opening (211) being located on the first housing (21) of the working component (2);
a handheld component (1), detachably connected to the working component (2); and
a first communication assembly (422), located in the first housing (21) of the working component (2);
wherein the coupling component (32) further comprises:
a second communication assembly (322), located at a proximal end of the coupling casing (321),
wherein the second communication assembly (322) and the first communication assembly (422) are engaged with each other in a plugged-in manner so as to communicate with each other;
wherein the second communication assembly (322) comprises:
a communication casing (3221), mounted on the coupling casing (321) and is configured to be rotatable relative to the coupling casing (321); and
an electrical terminal (3222), disposed within the communication casing (3221).

5. The surgical apparatus according to claim 4, wherein the working component (2) further comprises:
a driving rod (23), disposed in the first housing (21), wherein the driving rod (23) is configured to be in a reciprocating motion along a direction parallel to a central axis (B) of the rotary head (22).

6. The surgical apparatus according to claim 5, wherein the shaft assembly further comprises:
a clip-cartridge assembly (33), located in the firing sleeve (31) and configured to provide a ligating clip (332) and to deliver the ligating clip (332); and
a rod assembly (34), comprising a pushing rod (341) connected to the clip-cartridge assembly (33) and a firing rod (342) configured to push the firing sleeve (31), wherein the firing rod (342) is provided with a hollow portion, the pushing rod (341) is disposed within the hollow portion and is configured to be slidable relative to the firing rod (342) ; the pushing rod (341) is configured to move in a first direction (+A) parallel to the central axis (B) of the rotary head (22), so that the clip-cartridge assembly (33) delivers the ligating clip (332), and the pushing rod (341) is configured to continues to move in the first direction (+A) against the firing rod (342) so that the firing sleeve (31) moves in the first direction (+A);
wherein the second communication assembly (322) further comprises:
a supporting member (3223), located in the communication casing (3221) and configured to support the electrical terminal (3222), wherein the supporting member (3223) is provided with a second through hole (3227);
wherein the driving rod (23) is configured to pass through the second through hole (3227) to be abutted against the pushing rod (341) so that the pushing rod (341) moves in the first direction (+A).

7. The surgical apparatus according to claim 6, wherein the firing sleeve (31) comprising a flange (440), the firing rod (342) is provided with a bump (3422) and a first restoration member (35), wherein the first restoration member (35) is disposed between the bump (3422) and the flange (440) so that the firing rod (342) is restored to an original position.

8. The surgical apparatus according to claim 7, wherein one end of the pushing rod (341) is provided with a stop block (3411), and a second restoration member (36) is disposed between the stop block (3411) and the bump (3422) so that the pushing rod (341) is restored to an original position.

9. The surgical apparatus according to any one of claims 1-8, wherein:
the adapter comprises:
a working component (102); and
a handheld component (101), detachably connected to the working component (102);
the shaft assembly (103) is configured to insert into the working component (102) and the shaft assembly (103) comprises:
a clip-cartridge assembly (131), located in the firing sleeve (31) and configured to provide a ligating clip (1312) and to deliver the ligating clip (1312); and
a clip jaw (132);
the surgical apparatus (10) further comprises:
an attitude sensing assembly (30); and
a control assembly (40), electrically connected with the attitude sensing assembly (30);
wherein the attitude sensing assembly (30) is configured to acquire attitude data of the surgical apparatus (10) and send the attitude data to the control assembly (40); the control assembly (40) is configured to determine whether to activate an automatic clip-delivery mode based on the attitude data and determine whether the ligating clip (1312) is placed on the clip jaw (132);
wherein the clip-cartridge assembly (131) automatically delivers the ligating clip (1312) to the clip jaw (132), if it's determined to activate the automatic clip-delivery mode and determined that the ligating clip (1312) is not placed on the clip jaw (132).

10. The surgical apparatus according to claim 9, wherein the attitude sensing assembly (30) comprises an acceleration sensor, and the attitude data comprises acceleration data of the surgical apparatus (10).

11. The surgical apparatus according to claim 10, wherein the control assembly (40) is further configured to:
determine to activate the automatic clip-delivery mode, if a variation amount of acceleration data within a first preset time period is matched with a first template variation rule, in the case that a number of the ligating clip (1312) having been delivered to the clip jaw (132) is zero;
wherein the first template variation rule is established based on a variation amount of acceleration data in a starting process of a surgery operated by the surgical apparatus (10);
wherein the first template variation rule is that, a variation amount of displacement, along a central axis of the shaft assembly (103) and towards the clip jaw (132), within the first preset time period is greater than a first preset value.

12. The surgical apparatus according to claim 10, wherein the control assembly (40) is further configured to:
determine to activate the automatic clip-delivery mode, if a variation amount of acceleration data within a second preset time period is not matched with a second template variation rule, in the case that a number of the ligating clip (1312) having been delivered to the clip jaw (132) is greater than zero;
wherein the second template variation rule is established based on a variation amount of acceleration data in an ending process of a surgery operated by the surgical apparatus (10);
wherein the second template variation rule is that, a variation amount of displacement, along a central axis of the shaft assembly (103) and towards the handheld component (101), within the second preset time period is greater than a second preset value.

13. The surgical apparatus according to any one of claims 9-12, further comprising:
a driving assembly (111), disposed in the handheld component (101); and
a transmission assembly and a pushing rod (112), both disposed in the working component, wherein the transmission assembly is connected to the driving assembly (111), and the pushing rod (112) is connected to the transmission assembly;
wherein the pushing rod (112) is configured to in axially reciprocating motion under a drive of the driving assembly (111) and the transmission assembly, so as to push the clip-cartridge assembly (131) to automatically deliver the ligating clip (1312) to the clip jaw (132).

14. The surgical apparatus according to claim 13, further comprising:
a motor circuit (113), disposed in the handhold component (101), wherein one end of the motor circuit (113) is connected to the control assembly (40), and the other end of the motor circuit (113) is connected to the driving assembly (111), the motor circuit (113) is configured to receive a control signal from the control assembly (40) and to control a movement of the driving assembly (111) according to the control signal; and
an automatic clip-delivery switch, connected to the control assembly (40);
wherein the automatic clip-delivery switch is configured to send an automatic clip-delivery signal to the control assembly (40) in the case that the automatic clip-delivery switch is triggered;
wherein the control assembly (40) is further configured to, upon receiving the automatic clip-delivery signal, determine to activate the automatic clip-delivery mode and send an automatic clip-delivery control signal to the motor circuit (113), if a variation amount of acceleration data within a second preset time period is not matched with a second template variation rule; and
wherein the motor circuit (113) is configured to control a movement of the driving assembly (111) according to the automatic clip-delivery control signal, so that the transmission assembly drives the pushing rod (112) to push the clip-cartridge assembly (131) to automatically deliver the ligating clip (1312) to the clip jaw (132).

## Patentansprüche

1. Chirurgische Vorrichtung, umfassend:
ein Passstück, umfassend:
ein Gehäuse, umfassend eine Öffnung (211), die an einem distalen Ende des Gehäuses bereitgestellt ist; und
einen Rotationskopf (22), der über die Öffnung (211) des Gehäuses geschoben ist und dazu ausgestaltet ist, relativ zum Gehäuse drehbar zu sein, wobei der Rotationskopf (22) ein erstes Durchgangsloch (266) und ein im ersten Durchgangsloch (266) angeordnetes erstes Führungselement (222) umfasst;
eine Wellenbaugruppe (3), die abnehmbar mit dem Passstück verbunden ist und Folgendes umfasst:
eine Abschusshülse (31); und
eine Kopplungskomponente (32), die an einem Ende der Abschusshülse (31) angeordnet ist, wobei die Kopplungskomponente (32) dazu ausgestaltet ist, in die Öffnung (211) des Gehäuses eingeführt zu werden, nachdem sie das erste Durchgangsloch (266) des Rotationskopfes (22) passiert hat, wobei die Kopplungskomponente (32) ein Kopplungsgehäuse (321) und ein am Kopplungsgehäuse (321) angeordnetes zweites Führungselement (323) umfasst, **dadurch gekennzeichnet, dass**
das erste Führungselement (222) einen ersten Keilabschnitt umfasst, der sich in eine erste Richtung (+A) parallel zu einer Mittelachse (B) des Rotationskopfes (22) verjüngt, und das zweite Führungselement (323) einen zweiten Keilabschnitt umfasst, der sich in eine zweite Richtung (-A) parallel zur Mittelachse (B) des Rotationskopfes (22) verjüngt, wobei die erste Richtung (+A) der zweiten Richtung (-A) entgegengesetzt ist und das erste Führungselement (222) und das zweite Führungselement (323) in einer Schnappverbindung ineinander eingerastet sind, um sowohl den Rotationskopf (22) als auch die Wellenbaugruppe (3) in einer arretierten Position zu halten.

2. Chirurgische Vorrichtung nach Anspruch 1, wobei:
der Rotationskopf (22) dazu ausgestaltet ist, das Kopplungsgehäuse (321) anzutreiben, sich um die Mittelachse (B) des Rotationskopfes (22) zu drehen, und die Kopplungskomponente (32) dazu ausgestaltet ist, in den Rotationskopf (22) entlang einer Richtung parallel zur Mittelachse (B) eingeführt zu werden.

3. Chirurgische Vorrichtung nach Anspruch 1 oder 2, wobei:
das erste Führungselement (222) mindestens zwei erste Vorsprünge (2221) umfasst und die mindestens zwei ersten Vorsprünge (2221) voneinander beabstandet sind; und
sich das zweite Führungselement (323) auf einer Oberfläche des Kopplungsgehäuses (321) befindet und einen zweiten Vorsprung (3231) umfasst, der zwischen den mindestens zwei ersten Vorsprüngen (2221) in einer Schnappverbindung eingerastet ist.

4. Chirurgische Vorrichtung nach einem der Ansprüche 1-3,
wobei das Passstück Folgendes umfasst:
eine Arbeitskomponente (2), die ein erstes Gehäuse (21) aufweist, wobei sich die Öffnung (211) auf dem ersten Gehäuse (21) der Arbeitskomponente (2) befindet;
eine Handkomponente (1), die abnehmbar mit der Arbeitskomponente (2) verbunden ist; und
eine erste Kommunikationsbaugruppe (422), die sich im ersten Gehäuse (21) der Arbeitskomponente (2) befindet;
wobei die Kopplungskomponente (32) ferner Folgendes umfasst:
eine zweite Kommunikationsbaugruppe (322), die sich an einem proximalen Ende des Kopplungsgehäuses (321) befindet,
wobei die zweite Kommunikationsbaugruppe (322) und die erste Kommunikationsbaugruppe (422) in einer Steckverbindung ineinander eingerastet sind, um miteinander zu kommunizieren;
wobei die zweite Kommunikationsbaugruppe (322) Folgendes umfasst:
ein Kommunikationsgehäuse (3221), das an dem Kopplungsgehäuse (321) montiert ist und dazu ausgestaltet ist, relativ zum Kopplungsgehäuse (321) drehbar zu sein; und
einen elektrischen Anschluss (3222), der innerhalb des Kommunikationsgehäuses (3221) angeordnet ist.

5. Chirurgische Vorrichtung nach Anspruch 4, wobei die Arbeitskomponente (2) ferner Folgendes umfasst:
eine Antriebsstange (23), die im ersten Gehäuse (21) angeordnet ist, wobei die Antriebsstange (23) dazu ausgestaltet ist, sich entlang einer Richtung parallel zu einer Mittelachse (B) des Rotationskopfes (22) hin- und herzubewegen.

6. Chirurgische Vorrichtung nach Anspruch 5, wobei die Wellenbaugruppe ferner Folgendes umfasst:
eine Klammerpatronenbaugruppe (33), die sich in der Abschusshülse (31) befindet und dazu ausgestaltet ist, eine Ligaturklammer (332) bereitzustellen und die Ligaturklammer (332) abzugeben; und
eine Stangenbaugruppe (34), die eine Schubstange (341), die mit der Klammerpatronenbaugruppe (33) verbunden ist, und eine Abschussstange (342), die dazu ausgestaltet ist, die Abschusshülse (31) anzuschieben, umfasst, wobei die Abschussstange (342) mit einem Hohlabschnitt versehen ist, die Schubstange (341) innerhalb des Hohlabschnitts angeordnet ist und dazu ausgestaltet ist, relativ zur Abschussstange (342) verschiebbar zu sein; die Schubstange (341) dazu ausgestaltet ist, sich in eine erste Richtung (+A) parallel zur Mittelachse (B) des Rotationskopfes (22) zu bewegen, sodass die Klammerpatronenbaugruppe (33) die Ligaturklammer (332) abgibt, und die Schubstange (341) dazu ausgestaltet ist, sich weiterhin in die erste Richtung (+A) gegen die Abschussstange (342) zu bewegen, sodass sich die Abschusshülse (31) in die erste Richtung (+A) bewegt;
wobei die zweite Kommunikationsbaugruppe (322) ferner Folgendes umfasst:
ein Tragelement (3223), das sich im Kommunikationsgehäuse (3221) befindet und dazu ausgestaltet ist, den elektrischen Anschluss (3222) zu tragen, wobei das Tragelement (3223) mit einem zweiten Durchgangsloch (3227) versehen ist;
wobei die Antriebsstange (23) dazu ausgestaltet ist, das zweite Durchgangsloch (3227) zu passieren, um gegen die Schubstange (341) zu stoßen, sodass sich die Schubstange (341) in die erste Richtung (+A) bewegt.

7. Chirurgische Vorrichtung nach Anspruch 6, wobei die Abschusshülse (31) einen Flansch (440) umfasst, die Abschussstange (342) mit einer Erhebung (3422) und einem ersten Zurückstellelement (35) versehen ist, wobei das erste Zurückstellelement (35) zwischen der Erhebung (3422) und dem Flansch (440) angeordnet ist, sodass die Abschussstange (342) in eine ursprüngliche Position zurückgestellt wird.

8. Chirurgische Vorrichtung nach Anspruch 7, wobei ein Ende der Schubstange (341) mit einem Anschlagblock (3411) versehen ist und ein zweites Zurückstellelement (36) zwischen dem Anschlagblock (3411) und der Erhebung (3422) angeordnet ist, sodass die Schubstange (341) in eine ursprüngliche Position zurückgestellt wird.

9. Chirurgische Vorrichtung nach einem der Ansprüche 1-8, wobei:
das Passstück Folgendes umfasst:
eine Arbeitskomponente (102); und
eine Handkomponente (101), die abnehmbar mit der Arbeitskomponente (102) verbunden ist;
die Wellenbaugruppe (103) dazu ausgestaltet ist, in die Arbeitskomponente (102) eingeführt zu werden, und die Wellenbaugruppe (103) Folgendes umfasst:
eine Klammerpatronenbaugruppe (131), die sich in der Abschusshülse (31) befindet und dazu ausgestaltet ist, eine Ligaturklammer (1312) bereitzustellen und die Ligaturklammer (1312) abzugeben; und
eine Klemmbacke (132);
wobei die chirurgische Vorrichtung (10) ferner Folgendes umfasst:
eine Lagesensorbaugruppe (30); und
eine Steuerungsbaugruppe (40), die mit der Lagesensorbaugruppe (30) elektrisch verbunden ist;
wobei die Lagesensorbaugruppe (30) dazu ausgestaltet ist, Lagedaten der chirurgischen Vorrichtung (10) zu erfassen und die Lagedaten an die Steuerungsbaugruppe (40) zu senden; die Steuerungsbaugruppe (40) dazu ausgestaltet ist, basierend auf den Lagedaten zu bestimmen, ob ein automatischer Klammerabgabemodus aktiviert wird, und zu bestimmen, ob die Ligaturklammer (1312) auf der Klemmbacke (132) platziert wird;
wobei die Klammerpatronenbaugruppe (131) die Ligaturklammer (1312) automatisch an die Klemmbacke (132) abgibt, wenn bestimmt wird, dass der automatische Klammerabgabemodus aktiviert wird, und bestimmt wird, dass die Ligaturklammer (1312) nicht auf der Klemmbacke (132) platziert wird.

10. Chirurgische Vorrichtung nach Anspruch 9, wobei die Lagesensorbaugruppe (30) einen Beschleunigungssensor umfasst und die Lagedaten Beschleunigungsdaten der chirurgischen Vorrichtung (10) umfassen.

11. Chirurgische Vorrichtung nach Anspruch 10, wobei die Steuerungsbaugruppe (40) ferner dazu ausgestaltet ist, um:
zu bestimmen, ob in dem Fall, dass eine Anzahl der an die Klemmbacke (132) abgegebenen Ligaturklammern (1312) null ist, der automatische Klammerabgabemodus aktiviert wird, wenn eine Variationsmenge an Beschleunigungsdaten innerhalb eines ersten voreingestellten Zeitraums mit einer ersten Vorlagenvariationsregel abgeglichen wird;
wobei die erste Vorlagenvariationsregel basierend auf einer Variationsmenge an Beschleunigungsdaten in einem Startprozess einer von der chirurgischen Vorrichtung (10) vorgenommenen Operation festgelegt wird;
wobei die erste Vorlagenvariationsregel besagt, dass eine Variationsmenge an Verschiebung entlang einer Mittelachse der Wellenbaugruppe (103) und in Richtung der Klemmbacke (132) innerhalb des ersten voreingestellten Zeitraums größer ist als ein erster voreingestellter Wert.

12. Chirurgische Vorrichtung nach Anspruch 10, wobei die Steuerungsbaugruppe (40) ferner dazu ausgestaltet ist, um:
zu bestimmen, ob in dem Fall, dass eine Anzahl der an die Klemmbacke (132) abgegebenen Ligaturklammern (1312) größer als null ist, der automatische Klammerabgabemodus aktiviert wird, wenn eine Variationsmenge an Beschleunigungsdaten innerhalb eines zweiten voreingestellten Zeitraums mit einer zweiten Vorlagenvariationsregel abgeglichen wird;
wobei die zweite Vorlagenvariationsregel basierend auf einer Variationsmenge an Beschleunigungsdaten in einem Abschlussprozess einer von der chirurgischen Vorrichtung (10) vorgenommenen Operation festgelegt wird;
wobei die zweite Vorlagenvariationsregel besagt, dass eine Variationsmenge an Verschiebung entlang einer Mittelachse der Wellenbaugruppe (103) und in Richtung der Handkomponente (101) innerhalb des zweiten voreingestellten Zeitraums größer ist als ein zweiter voreingestellter Wert.

13. Chirurgische Vorrichtung nach einem der Ansprüche 9-12, ferner umfassend:
eine Antriebsbaugruppe (111), die in der Handkomponente (101) angeordnet ist; und
eine Übertragungsbaugruppe und eine Schubstange (112), die beide in der Arbeitskomponente angeordnet sind, wobei die Übertragungsbaugruppe mit der Antriebsbaugruppe (111) verbunden ist und die Schubstange (112) mit der Übertragungsbaugruppe verbunden ist;
wobei die Schubstange (112) dazu ausgestaltet ist, sich unter einem Antrieb der Antriebsbaugruppe (111) und der Übertragungsbaugruppe axial hin- und herzubewegen, um die Klammerpatronenbaugruppe (131) anzuschieben, um die Ligaturklammer (1312) automatisch an die Klemmbacke (132) abzugeben.

14. Chirurgische Vorrichtung nach Anspruch 13, ferner umfassend:
eine Motorschaltung (113), die in der Handkomponente (101) angeordnet ist, wobei ein Ende der Motorschaltung (113) mit der Steuerungsbaugruppe (40) verbunden ist und das andere Ende der Motorschaltung (113) mit der Antriebsbaugruppe (111) verbunden ist, die Motorschaltung (113) dazu ausgestaltet ist, ein Steuersignal von der Steuerungsbaugruppe (40) zu empfangen und eine Bewegung der Antriebsbaugruppe (111) gemäß dem Steuersignal zu steuern; und
einen automatischen Klammerabgabeschalter, der mit der Steuerungsbaugruppe (40) verbunden ist;
wobei der automatische Klammerabgabeschalter dazu ausgestaltet ist, in dem Fall, dass der automatische Klammerabgabeschalter ausgelöst wird, ein automatisches Klammerabgabesignal an die Steuerungsbaugruppe (40) zu senden;
wobei die Steuerungsbaugruppe (40) ferner dazu ausgestaltet ist, bei Empfang des automatischen Klammerabgabesignals zu bestimmen, ob der automatische Klammerabgabemodus aktiviert wird, und ein automatisches Klammerabgabesteuersignal an die Motorschaltung (113) zu senden, wenn eine Variationsmenge an Beschleunigungsdaten innerhalb eines zweiten voreingestellten Zeitraums nicht mit einer zweiten Vorlagenvariationsregel abgeglichen wird; und
wobei die Motorschaltung (113) dazu ausgestaltet ist, eine Bewegung der Antriebsbaugruppe (111) gemäß dem automatischen Klammerabgabesteuersignal zu steuern, sodass die Übertragungsbaugruppe die Schubstange (112) antreibt, die Klammerpatronenbaugruppe (131) anzuschieben, um die Ligaturklammer (1312) automatisch an die Klemmbacke (132) abzugeben.

## Revendications

1. Appareil chirurgical comprenant :
un adaptateur comprenant :
un boîtier comprenant une ouverture (211) pourvue à une extrémité distale du boîtier ; et
une tête rotative (22) emmanchée sur l'ouverture (211) du boîtier et conçue pour être rotatoire par rapport au boîtier, la tête rotative (22) comprenant un premier trou débouchant (266) et un premier organe de guidage (222) disposé dans le premier trou débouchant (266) ;
un ensemble à arbre (3) connecté de manière séparable à l'adaptateur et comprenant :
une gaine de décharge (31) ; et
une pièce d'accouplement (32) disposée à une extrémité de la gaine de décharge (31), la pièce d'accouplement (32) étant conçue pour s'insérer dans l'ouverture (211) du boîtier après passage au travers du premier trou débouchant (266) de la tête rotative (22), la pièce d'accouplement (32) comprenant un boîtier d'accouplement (321) et un second organe de guidage (323) disposé sur le boîtier d'accouplement (321), **caractérisé en ce que**
le premier organe de guidage (222) comprend une première partie biseautée qui est effilée dans une première direction (+A) parallèle à un axe central (B) de la tête rotative (22) et le second organe de guidage (323) comprend une seconde partie biseautée qui est effilée dans une seconde direction (-A) parallèle à l'axe central (B) de la tête rotative (22), la première direction (+A) étant opposée à la seconde direction (-A) et le premier organe de guidage (222) et le second organe de guidage (323) étant en prise par encliquetage l'un avec l'autre de manière à maintenir à la fois la tête rotative (22) et l'ensemble à arbre (3) dans une position verrouillée.

2. Appareil chirurgical selon la revendication 1, où :
la tête rotative (22) est conçue pour entraîner la rotation du boîtier d'accouplement (321) autour de l'axe central (B) de la tête rotative (22), et la pièce d'accouplement (32) est conçue pour s'insérer dans la tête rotative (22) le long d'une direction parallèle à l'axe central (B).

3. Appareil chirurgical selon la revendication 1 ou 2, où :
le premier organe de guidage (222) comprend au moins deux premières protubérances (2221) et les au moins deux premières protubérances (2221) sont séparées l'une de l'autre ; et
le second organe de guidage (323) est localisé sur une surface du boîtier d'accouplement (321) et comprend une seconde protrusion (3231) qui est en prise par encliquetage entre les au moins deux première protubérances (2221).

4. Appareil chirurgical selon l'une quelconque des revendications 1-3,
où l'adaptateur comprend :
une pièce travaillante (2) comportant un premier logement (21), l'ouverture (211) étant localisée sur le premier logement (21) de la pièce travaillante (2) ;
une pièce à main (1) connectée de manière séparable à la pièce travaillante (2) ; et
un premier ensemble de communication (422) localisé dans le premier logement (21) de la pièce travaillante (2) ;
où la pièce d'accouplement (32) comprend en outre :
un second ensemble de communication (322) localisé à une extrémité proximale du boîtier d'accouplement (321),
où le second ensemble de communication (322) et le premier ensemble de communication (422) sont en prise l'un avec l'autre par enfichage de manière à communiquer l'un avec l'autre ;
où le second ensemble de communication (322) comprend :
un boîtier de communication (3221) monté sur le boîtier d'accouplement (321) et conçu pour être rotatoire par rapport au boîtier d'accouplement (321) ; et
une borne électrique (3222) disposée dans le boîtier de communication (3221).

5. Appareil chirurgical selon la revendication 4, où la pièce travaillante (2) comprend en outre :
une tige d'entraînement (23) disposée dans le premier logement (21), où la tige d'entraînement (23) est conçue pour présenter un mouvement de va-et-vient le long d'une direction parallèle à l'axe central (B) de la tête rotative (22).

6. Appareil chirurgical selon la revendication 5, où l'ensemble à arbre comprend en outre :
un ensemble à cartouche de clips (33) localisé dans la gaine de décharge (31) et conçu pour fournir un clip de ligature (332) et larguer le clip de ligature (332) ; et
un ensemble à tige (34) comprenant une tige de poussée (341) connectée à l'ensemble à cartouche de clips (33) et une tige de décharge (342) conçue pour exercer une pression sur la gaine de décharge (31), où la tige de décharge (342) est pourvue d'une partie creuse, la tige de poussée (341) étant disposée dans la partie creuse et conçue de manière à pouvoir coulisser par rapport à la tige de décharge (342) ; la tige de poussée (341) étant conçue pour se déplacer dans une première direction (+A) qui est parallèle à l'axe central (B) de la tête rotative (22), de sorte que l'ensemble à cartouche de clips (33) largue le clip de ligature (332), et la tige de poussée (341) étant conçue pour continuer à se déplacer dans la première direction (+A) contre la tige de décharge (342), de sorte que la gaine de décharge (31) se déplace dans la première direction (+A) ;
où le second ensemble de communication (322) comprend en outre :
un élément de soutien (3223) localisé dans le boîtier de communication (3221) et conçu pour soutenir la borne électrique (3222), où l'élément de soutien (3223) est pourvu d'un second trou débouchant (3227) ;
où la tige d'entraînement (23) est conçue pour passer au travers du second trou débouchant (3227) de manière à être en butée contre la tige de poussée (341), de sorte que la tige de poussée (341) se déplace dans la première direction (+A).

7. Appareil chirurgical selon la revendication 6, où la gaine de décharge (31) comprend une bride (440) et où la tige de décharge (342) est pourvue d'une bosse (3422) et d'un premier organe de restauration (35), où le premier organe de restauration (35) est disposé entre la bosse (3422) et la bride (440), de sorte que la tige de décharge (342) est ramenée à une position originelle.

8. Appareil chirurgical selon la revendication 7, où une extrémité de la tige de poussée (341) est pourvue d'un bloc d'arrêt (3411) et où un second organe de restauration (36) est disposé entre le bloc d'arrêt (3411) et la bosse (3422), de sorte que la tige de poussée (341) est ramenée à une position originelle.

9. Appareil chirurgical selon l'une quelconque des revendications 1-8, où l'adaptateur comprend :
une pièce travaillante (102) ; et
une pièce à main (101) connectée de manière séparable à la pièce travaillante (102) ; l'ensemble à arbre (103) étant conçu pour s'insérer dans la pièce travaillante (102) et l'ensemble à arbre (103) comprend :
un ensemble à cartouche de clips (131) localisé dans la gaine de décharge (31) et conçu pour fournir un clip de ligature (1312) et larguer le clip de ligature (1312) ; et
une mâchoire de serrage des clips (132) ;
l'appareil chirurgical (10) comprenant en outre :
un ensemble de détection d'attitude (30) ; et
un ensemble de commande (40) connecté électriquement à l'ensemble de détection d'attitude (30) ;
où l'ensemble de détection d'attitude (30) est conçu pour saisir des données concernant l'attitude de l'appareil chirurgical (10) et envoyer les données d'attitude à l'ensemble de commande (40) ; l'ensemble de commande (40) étant conçu pour établir s'il convient ou non d'activer un mode de largage automatique des clips sur la base des données d'attitude et pour déterminer si le clip de ligature (1312) est ou non en place sur la mâchoire de serrage des clips (132) ;
où l'ensemble à cartouche de clips (131) largue automatiquement le clip de ligature (1312) dans la mâchoire de serrage des clips (132) s'il est établi qu'il convient d'activer le mode de largage automatique des clips et s'il est déterminé que le clip de ligature (1312) n'est pas en place sur la mâchoire de serrage des clips (132).

10. Appareil chirurgical selon la revendication 9, où l'ensemble de détection d'attitude (30) comprend un capteur d'accélération et où les données d'attitude comprennent des données concernant l'accélération de l'appareil chirurgical (10).

11. Appareil chirurgical selon la revendication 10, où l'ensemble de commande (40) est en outre conçu pour :
opter pour activer le mode de largage automatique des clips si un niveau de variation des données d'accélération au cours d'une première période de temps prédéfinie concorde avec une première règle du modèle de variation dans le cas où un nombre de clips de ligature (1312) qui ont été largués dans la mâchoire de serrage des clips (132) est zéro ;
où la première règle du modèle de variation est établie sur la base d'un niveau de variation des données d'accélération lors d'un processus de mise en oeuvre d'une chirurgie effectuée avec l'appareil chirurgical (10) ;
où la première règle du modèle de variation veut qu'un niveau de variation du déplacement, le long d'un axe central de l'ensemble à arbre (103) et vers la mâchoire de serrage des clips (132), au cours de la première période de temps prédéfinie est supérieur à une première valeur prédéfinie.

12. Appareil chirurgical selon la revendication 10, où l'ensemble de commande (40) est en outre conçu pour :
opter pour activer le mode de largage automatique des clips si un niveau de variation des données d'accélération au cours d'une seconde période de temps prédéfinie ne concorde pas avec une seconde règle du modèle de variation dans le cas où un nombre de clips de ligature (1312) qui ont été largués dans la mâchoire de serrage des clips (132) est supérieur à zéro ;
où la seconde règle du modèle de variation est établie sur la base d'un niveau de variation des données d'accélération lors d'un processus de terminaison d'une chirurgie effectuée avec l'appareil chirurgical (10) ;
où la seconde règle du modèle de variation veut qu'un niveau de variation du déplacement, le long d'un axe central de l'ensemble à arbre (103) et vers la pièce à main (101), au cours de la seconde période de temps prédéfinie est supérieur à une seconde valeur prédéfinie.

13. Appareil chirurgical selon l'une quelconque des revendications 9-12, comprenant en outre :
un ensemble d'entraînement (111) positionné dans la pièce à main (101) ; et
un ensemble de transmission et une tige de poussée (112) l'un et l'autre positionnés dans la pièce travaillante, où l'ensemble de transmission est connecté à l'ensemble d'entraînement (111) et où la tige de poussée (112) est connectée à l'ensemble de transmission ;
où la tige de poussée (112) est conçue pour adopter un mouvement de va-et-vient sous l'action de l'ensemble d'entraînement (111) et de l'ensemble de transmission, de sorte que l'ensemble à cartouche de clips (131) largue automatiquement le clip de ligature (1312) dans la mâchoire de serrage des clips (132).

14. Appareil chirurgical selon la revendication 13, comprenant en outre :
un circuit moteur (113) disposé dans la pièce à main (101), où un terminal du circuit moteur (113) est connecté à l'ensemble de commande (40) et l'autre terminal du circuit moteur (113) est connecté à l'ensemble d'entraînement (111), le circuit moteur (113) étant conçu pour recevoir un signal de commande provenant de l'ensemble de commande (40) et pour contrôler un déplacement de l'ensemble d'entraînement (111) en fonction du signal de commande ; et
un commutateur de largage automatique des clips, connecté à l'ensemble de commande (40) ;
où le commutateur de largage automatique des clips est conçu pour envoyer un signal de largage automatique des clips à l'ensemble de commande (40) dans le cas où le commutateur de largage automatique des clips est déclenché ;
où l'ensemble de commande (40) est en outre conçu pour opter pour activer le mode de largage automatique des clips à la réception du signal de largage automatique des clips, et pour envoyer un signal de largage automatique des clips au circuit moteur (113) si un niveau de variation des données d'accélération au cours d'une seconde période de temps prédéfinie ne concorde pas avec une seconde règle du modèle de variation ; et
où le circuit moteur (113) est conçu pour contrôler un déplacement de l'ensemble d'entraînement (111) en fonction du signal de commande du largage automatique des clips de sorte que l'ensemble de transmission conduit la tige de poussée (112) à pousser l'ensemble à cartouche de clips (131) pour larguer automatiquement le clip de ligature (1312) dans la mâchoire de serrage des clips (132).
